# EUROPEAN PATENT SPECIFICATION

(11) **EP 4 148 424 B1**
(45) Date of publication and mention of the grant of the patent: **12.08.2026**
(21) Application number: 22460038.7
(22) Date of filing: 29.08.2022
(51) Int. Cl.: G01N 27/12

(54) **METHOD AND SYSTEM OF MEASURING ATMOSPHERIC GAS COMPONENTS USING RESISTANCE GAS SENSOR**
VERFAHREN UND SYSTEM ZUR MESSUNG VON ATMOSPHÄRISCHEN GASKOMPONENTEN UNTER VERWENDUNG EINES WIDERSTANDSGASSENSORS
PROCÉDÉ ET SYSTÈME DE MESURE DE COMPOSANTS DE GAZ ATMOSPHÉRIQUE À L'AIDE D'UN CAPTEUR DE GAZ À RÉSISTANCE

(30) Priority: 14.09.2021 PL 43894321
(43) Date of publication of application: 15.03.2023
(73) Proprietor: Politechnika Gdanska, 80-233 Gdansk (PL)
(72) Inventor: Smulko, Janusz, 81-198 Suchy Dwor (PL); Kwiatkowski, Andrzej, 83-101 Straszyn (PL); Drozdowska, Katarzyna, 80-178 Gdansk (PL)
(74) Representative: Pawlowska-Bajerska, Justyna

(56) References cited:
- JP-B2- 4 602 000
- KR-B1- 102 193 342
- US-A1- 2018 238 846
- KWIATKOWSKI ANDRZEJ ET AL: "Embedded gas sensing setup for air samples analysis", REVIEW OF SCIENTIFIC INSTRUMENTS, AMERICAN INSTITUTE OF PHYSICS, 2 HUNTINGTON QUADRANGLE, MELVILLE, NY 11747, vol. 92, no. 7, 6 July 2021 (2021-07-06), XP012257920, ISSN: 0034-6748, [retrieved on 20210706], DOI: 10.1063/5.0050445

## Description

The invention refers to a method and measurement system with resistance gas sensors for measurement of gas components - gases in the measurement environment around gas sensor of gases known as gas sensor, gaseous sensor - detectors, indicating direct current resistance changes under the impact of UV radiation. The photocatalytic effect present in the gas-sensing layer is monitored in the gas sensor that form two metal electrodes, between which the photocatalytic (gas-sensing) layer is located, whereby the photocatalytic layer is made of materials such as WO₃, TiO₂, NiO, graphene, carbon nanotubes, gold nanoparticles coated with organic materials, etc. The invention is used to determine the composition of the gas atmosphere surrounding the detector.

The parameters of the resistance gas sensor are determined by its operating temperature and exposure to ultraviolet (UV) radiation. The operating temperature of the sensor influences the rate of adsorption and desorption, which determines the sensitivity and selectivity of the sensor. UV radiation supplies additional energy, which in one of the observed mechanisms causes the formation of oxygen photoions, in which the energy bonded with the sensor surface material is significantly lower than in other oxygen ions present on that surface. The group of photoions increases the sensitivity of the gas sensor, in particular in the range of low concentrations of the detected gases, due to the lower bonding energy and potential interactions with some of the gases surrounding the sensor. Therefore, this phenomenon has an impact on the selectivity of the sensor. In Bouchikhi, B., Chludziviski, T, Saidi, T., Smulko, J, El Bari, N, Wen, H., & Ionescu, R. (2020). Formaldehyde detection with chemical gas sensors based on WO3 nanowires decorated with metal nanoparticles under dark conditions and UV light irradiation. Sensors and Actuators B: Chemical, 320, 128331*,* it was proved that the application of UV radiation has a similar influence on gas sensor properties as metal doping if the sensor is made with a WO₃ layer.

The technical problem in known resistance sensors for gas detection is their limited selectivity, which is often influenced by the operating temperature, doping with selected metals or exposure to UV radiation in the case of the materials that show a photocatalytic effect. Metal doping - addition is not possible during the operation of the sensor. Modulation of the sensor properties by temperature changes is used, however it has limited influence on the increase in the selectivity of the sensor and usually requires a relatively long time for the thermal stabilisation of the gas-sensing layer.
The resistance of a single resistance sensor changes under the influence of changes in the surrounding atmosphere. An identical change in resistance may be caused by the presence of multiple gases in different concentrations. Therefore, gas sensor arrays with selective responses to different gases are often used in practice. This solution increases the costs and energy consumption of the devices using this type of sensor array. For these reasons, it is advantageous to use a single resistance sensor, modulated to increase its sensitivity and selectivity to different gases. Practical applications most often involve a mixture of gases. For these reasons, the identification of different gases must be enabled by the simplest possible system with reduced energy required for operation (e.g. heating of the gas-sensing layer to obtain sensitivity to the set gas).

According to the invention, it was found that the combined used of UV radiation and operating temperature changes (changes of operating conditions of gaseous sensor) enabled a significant modification of the selectivity of the resistance gas sensor. It was found that at least two temperature changes are required, e.g. from room temperature to a higher temperature, combined with the exposure to UV radiation of selected wavelengths, whereas one or more wavelengths may be used, i.e. the wavelength may be changed. It should be noted that the two factors that change the properties of the gas sensor are partially independent, which offers greater potential for improved gas detection with one sensor than with two sensors, each modulated separately with UV radiation or operating temperature. This approach eliminates the necessity of using multiple resistance sensors, while maintaining a high efficiency of the detection of selected gases.

The system according to the invention is based on that a resistance gas sensor with photocatalytic properties, a source of the supply voltage of the heating device, e.g. the heater, and at least one source of UV radiation, e.g. a UV LED that form an electrical system with a measurement component recording the changes in sensor resistance at its particular operating points, determined by the UV radiation intensity and gas-sensing layer temperature. Therefore, the resistance measurement system and thus the system used to measure and obtain data on the gas composition of the measurement environment - gases, according to the invention, comprises the following components: the measurement component recording the changes in resistance, c.g. in the form of a separate device, e.g. an ohmmeter, connected to the gas sensor in the form of two metal electrodes with a gas-sensing photocatalytic layer between these electrodes, preferably made of materials such as WO₃, TiO₂, NiO, graphene, carbon nanopipes or gold nanoparticles coated with organic materials or their mixtures. The measurement is performed using the resistance measurement method in known technical method. The gas sensor (sensor of gases, gaseous sensor) is coupled with a heating device, preferably a heater, whereas the heating device is connected to the first source of voltage, of which the value determines the operating temperature of the sensor by calibration using a thermometer. Therefore, the calibration of the voltage will correspond to the set temperatures. Simultaneously with temperature changes, the gas-sensing layer of the gas sensor is exposed to at least one wavelength of UV radiation, preferably with variable intensity, generated by a source or sources of radiation connected to the second modulated source of voltage.

The method of measurement of resistance comprises several steps wherein the above-described system is formed and then introducing a simultaneous change of the operating conditions of the sensor by changing the temperature and the wavelength of the UV radiation directed to the gas-sensing layer of the sensor, whereas the temperature is changed at least twice and at least one UV wavelength is used. Therefore, the temperature surrounding the sensor is changed in the temperature ranges pre-determined based on experimental testing for the selected material and its assumed volume using a heating device, preferably in the range between the room temperature and 300°C, and UV radiation is applied to the gas-sensing layer. It is preferable to introduce a change in the UV wavelength. At the same time, the temperature is controlled by means of voltage, the UV radiation intensity is controlled by means of voltage, and the resistance is recorded during these changes. Preferably, the UV wavelength is changed at set intervals, usually from 400 nm to 250 nm, based on the volume of the gas-sensing layer and the surface exposed to UV radiation, which determine the response time of the sensor. The range and specific UV wavelength values are partially determined by the availability of the sources of radiation. The concentrations and types of gases in the surroundings of the gas sensor are determined by reading the recorded changes in the resistance of the gas sensor and analysis of the measurement using know methods, based on the assumed detection algorithm (e.g. neural networks, main components analysis, support vectors machine).

In the invention, a known resistance gas sensor is used, in the form of two metal electrodes with a gas-sensing photocatalytic layer placed between them, which is exposed to UV radiation light and subject to changes in the operating temperature. The UV radiation changes the properties of the sensor, mainly in the photocatalytic top layer, which is penetrated by the radiation. According to the invention, the gas-sensing layer is heated at the same time, which changes the temperature of the gas-sensing layer to the operating temperature by means of a heating device, e.g. a heater. According to the invention, it is proposed to modulate the operating temperature- selected factor of operating conditions of gas sensor, and apply UV radiation at the same time - next factor, by means of two connected, independent sources of power. Selection of the established periodic changes of at least the temperature or both parameters for the two methods of modulation of the sensor properties depends on the properties of the gas-sensing material and its volume, therefore the combined application of both modulated factors with different intervals results in a wide range of variability of sensor properties (selectivity and sensitivity) for particular gases. In addition, each of the factors modulating the properties of the resistance gas sensor has a potentially different impact on its properties, depending in particular on the concentration of the gases detected in the atmosphere.

The essence of the system according to the invention is based on that the resistance gas sensor with photocatalytic properties, the source of the supply voltage of the sensor heating device, e.g. the heater, and at least one source of UV radiation, e.g. a UV LED, form an electrical system with a measurement component recording the changes in sensor resistance at its particular operating points, determined by the UV radiation intensity and gas-sensing layer temperature. The direct current resistance is measured using, e.g., a dedicated ohmmeter OM or other measurement component, using the measurement of resistance by measurement of the voltage drop at the voltage divider system, which includes the gas sensor.

The subject of the invention is also method of measurement of the gas components of the atmosphere. The method is characterized in that it is combining factors modulating of operating conditions of the sensor - while it is performed at least two changes of the temperature surrounding the resistance gas sensor, the gas-sensing layer, and it is applied at least one UV radiation wavelength, while at the same time measuring the resistance between the two metal electrodes using a known method or known measurement devices. For the purposes of temperature changes, a heating device supplied from a modulated source of voltage is used, and for wavelength changes, a source or sources of UV radiation supplied from a modulated source of voltage is used.

The information, data, obtained by means of the invention-direct current resistance measurements-from the gas sensor with thus modulated properties provides the data enabling the identification of the composition of the atmosphere around the sensor, like when using an entire array of gas sensors. The advantage is that enough data is collected on the atmosphere surrounding the sensor with the benefit of the invention that is only using a single sensor in the measurement system, which reduces the energy consumption and problems resulting from the use of an array of gas sensors (higher complexity of the measurement system, sensor maintenance problems, higher energy consumption, etc.), at the expense of a longer measurement time required by the modulation in use.

The invention makes it possible to replace an array of gas sensors varying in sensitivity and selectivity with a single sensor with modulated properties and a system simplified to a single measurement channel. Determining the resistance under different operating conditions of the sensor makes it possible to change its selectivity according to the gases detected in the atmosphere surrounding the sensor and its sensitivity of response to particular gases, in different concentration ranges. The response recorded in the form of a time-based string of resistance values measured at the set modulation of temperature and UV radiation constitutes a response standard, characteristic of the composition of the atmosphere surrounding the sensor. The invention features high sensitivity and selectivity relative to the existing solutions, which makes it possible to use a single resistance gas sensor to detect multiple gases.

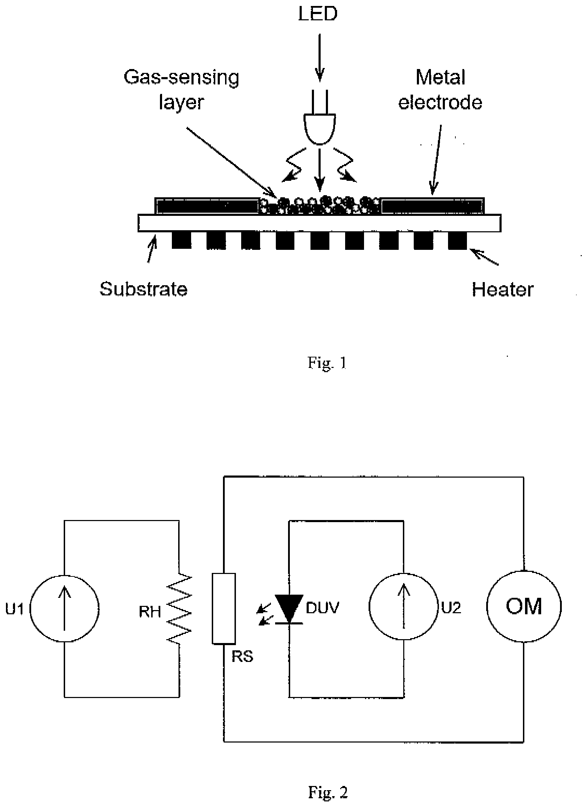

The subject of the invention is demonstrated by the embodiment and in Fig. 1 and Fig. 2. Fig. 1 presents the gas sensor with gas-sensing layer, terminals of two contact electrodes and components ensuring the modulation of sensor properties, while Fig. 2 presents an electrical diagram of the resistance measurement and sensor properties modulation system.

### Example

As shown in Fig. 1, the gas sensor consists of two metal electrodes placed symmetrically, in this embodiment made of a gold coating on a ceramic substrate, whereas the space between the metal electrodes is filled by a gas-sensing photocatalytic layer with the size of 1 mm x 1 mm and thickness of 800 nm, which in this example consists of NiO nanoparticles with a diameter of typically 6.4 nm.

Fig. 2 presents a measurement system recording changes in the resistance of the gas sensor RS. The system according to the invention is an electrical system including a gas sensor RS coupled with a heating device, the heater RH supplied from the first modulated source of voltage U1, a resistance meter, ohmmeter OM, and source of ultraviolet radiation DUV supplied from the second modulated source of voltage U2. In this example, the first source of voltage U1 is voltage in the range of 0-5 V and the second source of voltage U2 is modulated up to 12 V, with a current efficiency up to 150 mA. The gas sensor RS is connected with a heating device RH, a heater, by means of a common ceramic surface. The heater RH is made by screen printing with platinum applied on a ceramic substrate with a gas-sensing layer on the opposite side. The entire system is placed in the measurement environment, for this embodiment: in the atmosphere of example calibration gases (e.g. nitrogen dioxide, acetone), diluted in synthetic air to obtain the set concentration, different for the particular gases and not exceeding 30 ppm in each case. The measurement is performed using a dedicated ohmmeter OM, or by measurement of the resistance by measurement of the voltage drop at the voltage divider system-resistance measurement component, which includes the gas sensor. The heating device RH is connected to the first source of voltage U1, of which the value determines the operating temperature of the sensor by calibration using a thermometer. Therefore, the calibration of the voltage will correspond to the set temperatures.

During the measurement, the temperature of the heater RH of the gas sensor is changed in a specified range by means of modulation of the supply voltage of the first source of power U1. Simultaneously, the gas-sensing layer of the sensor is exposed to UV radiation with variable intensity, generated by a source of radiation DUV connected to the second modulated source of voltage U2. The change in temperature and the UV radiation wavelength are controlled by the setting of the value of voltage U1 in the range of 0 to 5 V and U2 in the range of 0 V to 12 V, which correspond to the pre-calibrated operating temperature of the sensor and radiation intensity of the UV light emitting diodes used with different wavelengths. In this embodiment, the temperature range around the gas-sensing layer is changed from room temperature to 300°C within 15 minutes, with a constant rate of temperature change using the heater RH with the power of 200 mW, while with regards to the UV radiation wavelength, sources of radiation DUV are used, e.g. commercially available diodes emitting the radiation with the maximum emitted power for the wavelengths of 365 nm and 275 nm. The value of 365 nm is maintained for 7 minutes and then changed to 275 nm. The UV wavelength may be changed by replacement with a diode emitting other wavelengths. However, a single UB wavelength may be used depending on the number of gas components to determine and the material used. For thus selected operating conditions, changes in resistance of the gas sensor are found, recorded between two electrodes using the ohmmeter OM within the total time of 15 minutes.

As a result of the use of the invention, by detecting differences in resistance waveforms under the same conditions of modulation of the gas-sensing layer, information is obtained-the time-based waveform characteristic of the composition of the atmosphere surrounding the sensor, constituting the input data vector for the gas detection algorithm in use.

In this example, the resistance is recorded in the range of 2.10⁵ Ω to 10·10⁵ Ω with the sensor placed in the vicinity of the above-mentioned calibration gases with concentrations up to 30 ppm. The composition of the environment surrounding the sensor is determined based on the selected detection algorithm, the support vector machine, so the input data for the detection algorithm is the vector of the recorded changes in resistance in time under the operating conditions of the sensor changing according to the pre-determined pattern. This method confirmed the composition of gas mixtures such as NO₂ and C₃H₆O.

## Claims

1. A system of measurement of gases comprising a gaseous sensor (RS) in the form of two metal electrodes placed symmetrically with a photocatalytic layer between wherein the electrodes are in a form of a gold coating on a ceramic substrate, whereas the space between the metal electrodes is filled by a gas-sensing photocatalytic layer made of NiO nanoparticles;
a heating device (RH) and at least one source of UV radiation (DUV);
a first modulated source of voltage (U1) supplying the heating device (RH);
a second modulated source of voltage (U2) supplying at least one source of UV radiation (DUV), and resistance measurement means configured to measure resistance between the two electrodes during gradually changing of temperature of the photocatalitic layer of the gas sensor (RS) by a time-varying voltage from the first modulated source of voltage (U1) and simultaneously expose the gas-sensing layer to UV radiation emitted from the source of UV radiation (DUV) supplied with voltage from the second modulated source of voltage (U2), **characterised in that** the system is configurated to generate at least two temperature changes with constant rate from room temperature up to 300°C and at least two UV radiation wavelength changes from 400 nm to 250 nm during one measurement cycle.

2. Method of measurement of gases comprising the steps of:
Providing the system according to claim 1; recording a direct current resistance by the system, while the operating conditions of the resistance gaseous sensor of the system are modulated such that one resistance measurement cycle comprises at least two temperature changes with constant rate from room temperature up to 300°C and at least two UV radiation wavelength changes from 400 nm to 250 nm.

## Patentansprüche

1. Ein System zur Messung von Gasen, umfassend einen Gassensor (RS) in Form von zwei symmetrisch angeordneten Metallelektroden mit einer dazwischenliegenden photokatalytischen Schicht, wobei die Elektroden als Goldbeschichtung auf einem Keramiksubstrat ausgebildet sind und der Raum zwischen den Metallelektroden mit einer gassensitiven photokatalytischen Schicht aus NiO-Nanopartikeln gefüllt ist;
eine Heizvorrichtung (RH) und mindestens eine UV-Strahlungsquelle (DUV);
eine erste modulierte Spannungsquelle (Ul) zur Versorgung der Heizvorrichtung (RH);
Eine zweite modulierte Spannungsquelle (U2), die mindestens eine UV-Strahlungsquelle (DUV) speist, und eine Widerstandsmesseinrichtung, die so konfiguriert ist, dass sie den Widerstand zwischen den beiden Elektroden während der allmählichen Änderung der Temperatur der photokatalytischen Schicht des Gassensors (RS) durch eine zeitlich veränderliche Spannung von der ersten modulierten Spannungsquelle (U1) misst und gleichzeitig die Gassensorschicht der UV-Strahlung aussetzt, die von der UV-Strahlungsquelle (DUV) emittiert wird, welche mit Spannung von der zweiten modulierten Spannungsquelle (U2) versorgt wird, **dadurch gekennzeichnet, dass** das System so konfiguriert ist, dass es während eines Messzyklus mindestens zwei Temperaturänderungen mit konstanter Rate von Raumtemperatur bis 300 °C und mindestens zwei UV-Strahlungswellenlängenänderungen von 400 nm bis 250 nm erzeugt.

2. Verfahren zur Messung von Gasen, umfassend die folgenden Schritte:
Bereitstellen des Systems nach Ansprüche 1; Aufzeichnen eines Gleichstromwiderstands durch das System, während die Betriebsbedingungen des Widerstandsgassensors des Systems so moduliert werden, dass ein Widerstandsmesszyklus mindestens zwei Temperaturänderungen mit konstanter Rate von Raumtemperatur bis 300 °C und mindestens zwei Änderungen der UV-Strahlungswellenlänge von 400 nm bis 250 nm umfasst.

## Revendications

1. Système de mesure de gaz comprenant un capteur de gaz (RS) constitué de deux électrodes métalliques disposées symétriquement, séparées par une couche photocatalytique. Ces électrodes sont réalisées par un revêtement d'or sur un substrat céramique, l'espace entre elles étant rempli par une couche photocatalytique sensible aux gaz, composée de nanoparticules de NiO;
un dispositif de chauffage (RH) et au moins une source de rayonnement UV (DUV);
une première source de tension modulée (Ul) alimentant le dispositif de chauffage (RH); une seconde source de tension modulée (U2) fournissant au moins une source de rayonnement UV (DUV), et des moyens de mesure de résistance configurés pour mesurer la résistance entre les deux électrodes lors du changement progressif de température de la couche photocatalytique du capteur de gaz (RS) par une tension variable dans le temps provenant de la première source de tension modulée (U1) et exposer simultanément la couche sensible au gaz au rayonnement UV émis par la source de rayonnement UV (DUV) alimentée par la tension de la seconde source de tension modulée (U2), **caractérisé en ce que** le système est configuré pour générer au moins deux changements de température à vitesse constante de la température ambiante jusqu'à 300 °C et au moins deux changements de longueur d'onde de rayonnement UV de 400 nm à 250 nm au cours d'un cycle de mesure.

2. Procédé de mesure de gaz comprenant les étapes suivantes:
Fourniture du système selon la revendication 1; enregistrement d'une résistance en courant continu par le système, tandis que les conditions de fonctionnement du capteur de gaz à résistance du système sont modulées de sorte qu'un cycle de mesure de résistance comprenne au moins deux variations de température à vitesse constante de la température ambiante jusqu'à 300 °C et au moins deux variations de longueur d'onde du rayonnement UV de 400 nm à 250 nm.
